# EUROPEAN PATENT APPLICATION

(11) **EP 2 110 441 A1**
(43) Date of publication of application: **21.10.2009**
(21) Application number: 09165173.7
(22) Date of filing: 30.04.2001
(51) Int. Cl.: C12Q 1/68, G01N 33/68

(54) **Method and probes for the genetic diagnosis of hemochromatosis**

(30) Priority: 02.05.2000 AT 7662000; 08.05.2000 AT 7992000
(62) Divisional of application: 01933906.8
(71) Applicant: ViennaLab Diagnostics GmbH, 1120 Vienna (AT)
(72) Inventor: Piperno, Alberto, 20124, Milano (IT); Gasparini, Paolo, Brandico (IT); Camaschella, Clara, 10128, Torino (IT); DE Villiers, Nico, 7580, Western Cape Province (ZA); Oberkanins, Christian, 1060, Vienna (AT); Kury, Friedrich, 1180, Vienna (AT)
(74) Representative: Redl, Gerda

(57) **Abstract**

A method to diagnose hemochromatosis includes examining a biological sample for the presence of a G → A mutation at nucleotide 506 and/or a G → T mutation at nucleotide 502 and/or of a G → C mutation at nucleotide 502 of the HFE cDNA sequence and/or by examining the biological sample for the presence of a C → G mutation at nucleotide 750 and/or a T → A mutation at nucleotide 515 and/or a frameshift mutation by insertion of a cytosine in a polyC tract at nucleotides 84-88 of the TFR2 cDNA sequence or examining a biological sample for the presence of respective amino acid substitutions. Probes according to the invention are capable of hybridizing with nucleic acids of a biological sample in a region corresponding to a region of the HFE or TFR2 cDNA sequence containing the above mentioned positions if respective mutations exist.

## Description

The invention relates to a method for the genetic diagnosis of hemochromatosis as well as to probes for the genetic diagnosis of hemochromatosis.

Hereditary Hemochromatosis (HH) /11/ is a common autosomal recessive disease characterized by a disorder of the iron metabolism in the human body leading to progressive iron overload. The excess iron is deposited in various organs, primarily liver, heart and pancreas, causing irreversible damages and diseases as liver cirrhosis, diabetes, arthritis, cardiomyopathies and premature death. With early diagnosis and treatment by therapeutic phlebotomy, organ damage can be completely prevented and life expectancy of patients is normal. As clinical symptoms of hemochromatosis often appear not till the age of 40, 50 or older, when irreversible damages of organs already may exist, a presymptomatic diagnosis is favourized.

A number of point mutations within a novel MHC class I-like gene, termed HFE (locus 6p), have been identified and related to HH /1,7,9/. This gene encodes a protein that interacts with transferrin receptor (TFRC) and negatively affects cellular iron uptake from transferrin /36/. In 1996, the HFE gene was identified and two mutations have been originally described, C282Y and H63D /1/. These two mutations are presently used for the genetic diagnosis of hemochromatosis.

H63D is a C→G mutation in codon 63, converting the amino acid 63 from histidine into aspartic acid. Because of its high frequency in the general population, its role in the diagnosis of the disease remains uncertain.

A much better criterion for the diagnosis of hemochromatosis is the C282Y mutation, found in 65 to 95% of the concerned patients. C282Y is a G → A mutation at nucleotide 845 of the HFE cDNA sequence that converts the amino acid 282 from cysteine into tyrosine. So most HH patients are homozygous for C282Y /1,13,14,15/. Among HH probands, compound heterozygotes C282Y/H63D account for about 5-7% /1,16/, H63D homozygotes are very rare and both have generally a mild form of hemochromatosis /17/. A minority of cases presenting with a HH phenotype are C282Y or H63D heterozygotes or neither C282Y or H63D. This appears to be more common in southern than in northern Europe /18,19/. That means the percentage of hemochromatosis patients not showing a C282Y mutation varies and is e.g. higher in Italy than in countries of Northern Europe. In a collaborative study including 188 Italian patients with HH-like phenotype, 28% were incompletely characterised with at least one chromosome without an assigned mutation /19/. In some of these patients the presence of other HFE mutations or other 6p-linked genetic determinants or the existence of other form of genetic iron overload unlinked to chromosome 6 have been suggested /19,20,21/. Three novel HFE mutations have been recently discovered in the heterozygous state in association with C282Y in single cases with classical HH: a splice site mutation (IVS3 + 1G → T) causing obligate skipping of exon 3 /22/, and two missense mutations (I105T and G93R) located in α1 domain that may affect the binding of the HFE protein to the transferrin receptor /23/. Moreover, the S65C missense mutation was found to be significantly enriched in HH chromosomes that were neither C282Y or H63D, suggesting that the S65C could be another variant contributing to a mild form of HH in association with C282Y or H63D /9/. Other missense mutations have been recently described but their role in the pathogenesis of HH is still unclear /7/.

While homozygozity for C282Y is observed in the majority of Caucasian HH patients and is well recognized as a cause for the disease, the diagnostic value of other HFE mutations is still under investigation. It should be of particular interest to define disease-causing mutations in those HH patients who are not homozygous for C282Y.

It therefore is important to find - apart from the known mutations - those further genetic causes in the HFE gene or other genes, which may lead to the excess of iron in the patients.

For that reason the problem of the present invention is to find further possibilities to diagnose genetic causes for hemochromatosis.

The problem is solved by examining a biological sample for the presence of a G → A mutation at nucleotide 506 and/or a G→T mutation at nucleotide 502 of the HFE cDNA sequence. Such mutations have been found during examinations of five unrelated Italian patients heterozygous for C282Y with the classical hemochromatosis phenotype. The mutations (nt 506 G→A or W169X and nt 502 G→T or E168X) were identified in exon 3 of the C282Y negative chromosome. The G→A mutation at nucleotide 506 causes the conversion of the corresponding amino acid 169 from tryptophane into a stop codon (W169X), as well as the G→T mutation at nucleotide 502 causes the conversion of the corresponding amino acid 168 from glutamic acid into a stop codon (E168X).

The problem is also solved by examining a biological sample for the presence of a G→C mutation at nucleotide 502 of the HFE cDNA sequence. This exon 3 mutation (nt 502 G→C or E168Q) had been identified in a 79-year old Caucasian female from South-Africa. She was also heterozygous for the H63D mutation and her serum ferritin was moderately elevated at the time of testing (242 ng/ml; ref. values: 12-119 ng/ml). She presented without noteworthy clinical symptoms, but had a family history of both heart disease and different types of cancer. The E168Q mutation was initially identified by single-strand conformation polymorphism (SSCP) analysis /7/ and subsequently confirmed by DNA sequencing.The G→C mutation at nucleotide 502 causes the conversion of the corresponding amino acid 168 from glutamic acid into glutamine (E168Q).

The problem is further solved by examining a biological sample for the presence of a C→G mutation at nucleotide 750 of the TFR2 cDNA sequence. This mutation in exon 6 (nt 750 C→G or Y250X) had been identified in six affected patients from two unrelated families of Sicilian origin. All of them were homozygous for Y250X. The C→G mutation at position 750 of the cDNA sequence replaces a tyrosine with a stop codon at amino acid 250 (Y250X). The position of this mutation lies in 7q22. And this locus will subsequently be termed HFE3 (HFE: locus 6p; HFE2: locus 1q - juvenile hemochromatosis /26/).

The role of TFR2 in iron metabolism is poorly understood. TFR2 lacks iron responsive elements and does not show the iron-dependent post-transcriptional regulation present in other key genes of iron metabolism. Two transcripts have been identified /37/. The α-transcript product is a transmembrane protein that is primarily expressed in liver /37/. The TFR2 β-transcript is the result of alternative splicing; its protein product may be an intracellular protein and, though widley distributed, is expressed at low levels. The Y250X mutation is located in a region shared by both transcripts. A phenotype of iron overload associated with the absence of a functional gene suggests that TFR2 is more likely involved in iron regulation (as is HFE) rather than in iron uptake. It is also unlikely that TFR2 is involved in iron export, because the examined patients attained iron depletion by phlebotomies, implying that excess iron can be mobilized from the liver. Although TFR2 is highly expressed in the erythroid K562 cell line /37/, none of the patients showed erythocyte abnormalities. Rather, they tolerated long-term phlebotomies without developing anaemia.

The problem is further solved by examining a biological sample for the presence of a T→A mutation at nucleotide 515 of the TFR2 cDNA sequence. This mutation in exon 4 (nt 515 T→A or M172K) had been found in a single patient with non-HFE hemochromatosis originating from central Italy (Lazio region). The T→A mutation at position 515 of the cDNA sequence results in a lysine for methionine substitution at amino acid 172 (M172K).

The problem is further solved by examining a biological sample for the presence of a frameshift mutation by insertion of a cytosine in a polyC tract at nucleotides 84 to 88 of the TFR2 cDNA sequence. This mutation in exon 2 (84-88 insC or E60X) had been identified in six affected patients of a large family from Campania in the area South of Naples. The cytosine insertion in the polyC tract at positions 84 to 88 of the TFR2 cDNA sequence results in a frameshift followed by a premature stop codon at amino acid 60 (E60X).

Preferably the biological sample is additionally examined for the presence of a of a G→A mutation at nucleotide 845 and/or a C→G mutation at nucleotide 187 of the HFE cDNA sequence, so that a diagnosis of hemochromatosis may take place even if only one of these known mutations is found.

The problem is further solved by examining a biological sample for the presence of nucleic acids coding for HFE products with a substitution of valine with methionine at amino acid 53 or 59 (V53M, V59M) and/or of histidine with aspartic acid at amino acid 63 (H63D) and/or of serine with cysteine at amino acid 65 (S65C) and/or of glutamine with histidine at amino acid 127 (Q127H) and/or of glutamic acid with glutamine or a stop codon at amino acid 168 (E168Q, E168X) and/or of thryptophan with a stop codon at amino acid 169 (W169X) and/or of cysteine with tyrosine at amino acid 282 (C282Y) and/or of glutamine with proline at amino acid 283 (Q283P) or examining the biological sample for the presence of a HFE gene product with a substitution of valine with methionine at amino acid 53 or 59 (V53M, V59M) and/or of histidine with aspartic acid at amino acid 63 (H63D) and/or of serine with cysteine at amino acid 65 (S65C) and/or of glutamine with histidine at amino acid 127 (Q127H) and/or of glutamic acid with glutamine or a stop codon at amino acid 168 (E168Q, E168X) and/or of thryptophan with a stop codon at amino acid 169 (W169X) and/or of cysteine with tyrosine at amino acid 282 (C282Y) and/or of glutamine with proline at amino acid 283 (Q283P).

The problem is further solved by examining a biological sample for the presence of nucleic acids coding for TFR2 products with a substitution of glutamic acid with a stop codon at amino acid 60 (E60X) and/or of methionine with a lysine at amino acid 172 (M172K) and/or of tyrosine with a stop codon at amino acid 250 (Y20X) or examining the biological sample for the presence of a TFR2 gene product with a substitution of glutamic acid with a stop codon at amino acid 60 (E60X) and/or of methionine with a lysine at amino acid 172 (M172K) and/or of tyrosine with a stop codon at amino acid 250 (Y20X).

The point mutations known from the state of art or according to the present invention and described above and the frameshift mutation of the present invention as described above cause certain amino acid substitutions and have been found with patients affected by hemochromatosis. The amino acid substitutions described above however may be created also by mutations different from the said known mutations or mutations according to the present invention and will never the less lead to an affection or a carrier status. Examining a biological sample for the presence of one or more of the above amino acid substitutions, will therefore give information whether the patient the biological sample originates from is affected by or is a carrier of hemochromatosis.

For the biological sample blood, biopsic tissue, smear from the oral cavity, amniotic fluid or the like may be used and the biological sample is subjected to known pretreatments according to the selected known examination procedure.

If the biological sample is also examined for the presence of nucleic acids not showing the mentioned mutations or amino acid substitutions in their HFE gene or their TFR2 gene or respective gene products, it may be proved, whether the mutations are heterozygous or homozygous.

The examination may be accomplished in a known manner by sequence analysis of the nucleic acids derived from the biological sample.

Or nucleic acids of the biological sample are brought into contact with at least one probe capable of hybridizing with a region of said nucleic acids corresponding to a region of the HFE cDNA sequence containing nucleotide 506 if a G→A mutation exists at position 506 of the HFE cDNA sequence and/or containing nucleotide 502 if a G→T mutation exists at position 502 of the HFE cDNA sequence and/or containing nucleotide 502 if a G→C mutation exists at position 502 of the HFE gene and/or capable of hybridizing with a region of said nucleic acids corresponding to a region of the TFR2 cDNA sequence containing nucleotide 750 if a C→G mutation exists at position 750 of the TFR2 cDNA sequence and/or containing nucleotide 515 if a T→A mutation exists at position 515 of the TFR2 cDNA sequence and/or containing nucleotides 84 to 88 if a frameshift mutation by insertion of a cytosine in a polyC tract at nucleotides 84 to 88 of the TFR2 cDNA sequence exists and an examination is accomplished whether respective hybridization products have been created. From the biological sample nucleic acids (genomic DNA and/or RNA, seperately or as a mixture) are may be made accessible using any of the respective methods known from the state of art.

If the biological sample is to be examined for the presence of the known C282Y and/or H63D mutation too, nucleic acids of the biological sample are also contacted with at least one further probe capable of hybridising with a region of said nucleic acids corresponding to a region of the HFE cDNA sequence containing nucleotide 845 if a G→A mutation exists at nucleotide 845 and/or nucleotide 187 if a C→G mutation exists at nucleotide 187 of the HFE cDNA sequence and an examination is accomplished whether respective hybridization products have been created.

In order to differentiate whether one or more mutations are heterozygous or homozygous, the nucleic acids of the sample are preferably brought also into contact with at least one further probe capable of hybridising with a region of said nucleic acids corresponding to a region of the HFE or TFR2 cDNA sequence containing at least one of said nucleotides if no mutation exists and an examination is accomplished whether respective hybridization products have been created.

A number of different methods are currently used for the analysis of mutations, among them restriction fragment length polymorphism (RFLP), PCR with sequence-specific primers (PCR-SSP), allelespecific oligonucleotide hybridization, single-strand conformation polymorphism (SSCP), denaturing gradient gel electrophoresis (DGGE), oligonucleotide ligation assay (OLA), real-time fluorescence PCR, and DNA sequencing /1,2,3,4,5,6,7,8,9/. If RNA is examined, this may be accomplished for example by "NASBA" or RNA is transcribed into cDNA by reverse transcripton, which is amplified thereafter by PCR and which is charcterized successively. All these known analysis methods may be used for the method for diagnosis of the present invention.

The probe for the diagnosis of hemochromatosis by means of mutations in the HFE gene according to the invention is capable of hybridising with nucleic acids of a biological sample in a region corresponding to a region of the HFE cDNA sequence containing nucleotide 506 if a G→A mutation exists at position 506 of the HFE cDNA sequence and/or containing nucleotide 502 if a G→T mutation exists at position 502 of the HFE cDNA sequence and/or containing nucleotide 502 if a G→C mutation exists at position 502 of the HFE cDNA sequence.

The probe for the diagnosis of hemochromatosis by means of mutations in the TFR2 gene according to the invention is capable of hybridising with nucleic acids of a biological sample in a region corresponding to a region of the TFR2 cDNA sequence containing nucleotide 750 if a C→G mutation exists at position 750 of the TFR2 cDNA sequence and/or containing nucleotide 515 if a T→A mutation exists at position 515 of the TFR2 cDNA sequence and/or containing nucleotides 84 to 88 if a frameshift mutation by insertion of a cytosine in a polyC tract at nucleotides 84 to 88 of the TFR2 cDNA sequence exists.

The following examples describe performance possibilities for the methods for the diagnosis of hemochromatosis according to the invention. The enclosed drawings help to illustrate the examples. The graphic illustration of Fig. 1 shows some possible staining patterns obtainable with DNAs carrying different mutant and wild-type HFE or TFR2 alleles using reverse hybridization as explained in example 2. As refers to the examination of four families in respect to HFE3 (Y250X, M172K, E60X) according to example 4 Fig. 2a, 2b, 2c and 2d show the pedigrees of the four families (filled symbols affected, open symbols unaffected). Fig.3a, 3b and 3c show sequencing chromatographs of sequences of the TFR2 gene of exon 2, spanning the C insertion which originates the E60X mutation, of exon 4 spanning the M172K mutation at position 515 and of exon 6 spanning the Y250X mutation at position 750. Fig.4 shows a schematic representation of TFR2 structure in the 5'region of the gene.

### EXAMPLE 1:

### DNA Isolation and Amplification:

DNA was isolated from anticoagulated blood using standard extraction methods /10/ or commercially available reagents (GenXtract DNA extraction system, ViennaLab). HFE gene exon 3 sequences were amplified in a PCR reaction using primers 5'-GGA TTG GAG AGC AGC AGA AC-3' and 5'-AAA GTC CAA CCA GGG ATT CC-3'. Each primer was used at a final concentration of 0.4µM in 1xPCR buffer containing 1.5mM Mg2+, 200µM dNTPs (Epicentre, Madison, WI) and 0.02 U/µl AmpliTaq DNA polymerase (PE Biosystems, Foster City, CA). A thermocycling program of 30 cycles (94°C for 15s, 58°C for 30s, and 72°C for 30s) was performed on the GeneAmp PCR System 2400 (PE Biosystems).

### Sequencing:

PCR products were purified with "Centricon-100" columns according to the manufacturers recommendations. Amplified and purified DNA was quantitated by UV-spectrophotometry and subsequently sequenced strictly following the protocol described in the "BigDye Terminator Cycle Sequencing Ready Reaction Kit - with AmpliTaq DNA Polymerase, FS" (PE Applied Biosystems) for the ABI Prism instrumentation (PE Applied Biosystems).

### EXAMPLE 2:

Hereafter it is reported about the successful application of reverse hybridization, to set up a screening assay for the detection of some known mutations and some of the HFE and TFR2 point mutations as well as the TFR2 frameshift mutation according to the invention. The test is based on multiplex DNA amplification and ready-to-use membrane teststrips, which contain oligonucleotide probes for each wild-type and mutated allele immobilized as an array of parallel lines. The test is rapid, easy to perform and accessible to automation on commercially available equipment, and by adding new probes the teststrip can easily be adapted to cover an increasing number of mutations.

### DNA Isolation and Amplification:

DNA was isolated from anticoagulated blood using standard extraction methods /10/ or commercially available reagents (GenXtract DNA extraction system, ViennaLab). HFE gene exon 2, 3 and 4 sequences and TFR2 gene exon 2, 4 and 6 sequences were amplified in a single, multiplex PCR reaction using 5'-biotinylated primers (/1,31,32/; table 3). Each primer was used at a final concentration of 0.4 µM in 1x PCR buffer including 1.5 mM Mg2+, 200 µM dNTPs (Epicentre, Madison WI), and 0.02 U/µl AmpliTaq DNA polymerase (PE Biosystems, Foster City, CA). A thermocycling program of 30 cycles (94°C for 15 s, 58°C for 30 s and 72°C for 30 s) was performed on the GeneAmp PCR System 2400 (PE Biosystems).

### Preparation of Teststrips:

The general principle of tailing and immobilizing sequence-specific hybridization probes was described previously /34/. Probes (16-24 mers) specific for the wild-type or mutant allele of each mutation were selected from the HFE and TFR2 databank sequences (GenBank accession numbers: HFE-Z92910, TFR2-AF067864) and synthesized using standard phosphoramidite chemistry. A 3'-poly(dT) tail (300-500 residues) was enzymatically added by incubating oligonucleotides (200 pmol) in the presence of 60 U terminal deoxynucleotidyl transferase (Amersham Pharmacia, Buckinghamshire, UK) and 150 nmol dTTP (Epicentre) for 8 h at 37°C. The reaction was terminated by adding EDTA to 10 mM, and tailing efficiency was monitored by agarose gel electrophoresis. Poly(dT)-tailed probes were applied onto nitrocellulose membrane sheets (Whatman, Maidstone, UK) as individual parallel lines of 1 mm width using a custom-made slot-blot apparatus, dried and fixed by overnight baking at 60°C. A 5'-biotinylated control oligonucleotide was included to allow performance control of the detection reagents. The membrane sheet was finally sliced into 3 mm ready-to-use teststrips.

### Reverse-Hybridization and Detection:

The entire hybridization and detection procedure was carried out in individual lanes of a thin-walled plastic incubation tray (Bio-Rad, Hercules, CA). Equal volumes (10 µl) of PCR product and denaturing solution (400 mM NaOH, 10 mM EDTA) were mixed and incubated at room temperature for 5 min. The alkaline reaction was neutralized with sodium phosphate buffer, pH 7, a teststrip was added and hybridization was carried out for 30 min. at 45°C in a shaking waterbath. After three stringent washes at 45°C, bound PCR fragments were detected using a streptavidin-alkaline phosphatase conjugate and color substrates (NBT/BCIP). Upon positive reaction, a purple staining was visible after 15 min. incubation at room temperature.

### Results:

A multiplex PCR system was established to obtain biotinylated DNA fragments spanning exons 2, 3 and 4 of the HFE gene and exons 2, 4 and 6 of the TFR2 gene in a single amplification reaction. To avoid potential mistyping of the C282Y mutation due to an intronic polymorphism (5569G→A) within the binding region of the original reverse primer of Feder et al. (1996)/1/ the exon 4 fragment was modified according to Jeffrey et al. (1999)/32/.

To develop the reverse-hybridization assay DNA samples of individuals known to carry the following exonic HFE mutations were collected: HFE: V53M, V59M, H63D, H63H, S65C, Q127H, E168Q, E168X, W169X, C282Y, Q283P; TFR2: E60X, M172K and Y250X (Table 1).

**Table 1**

| Line | Probe specificity | Mutation designation | Reference |
|---|---|---|---|
| 1 | (control) | | |
| 2 | nt 157 G→A exon 2 | HFE V53M | Villiers et al. /7/ |
| 3 | nt 175 G→A exon 2 | HFE V59M | Villiers et al. /7/ |
| 4 | nt 187 C→G exon 2 | HFE H63D | Feder et al. /1/ |
| 5 | nt 189 T→C exon 2 | HFE H63H | Villiers et al. /7/ |
| 6 | nt 193 A→T exon 2 | HFE S65C | Mura et al. /9/ |
| 7 | nt 381 A→C exon 3 | HFE Q127H | Villiers et al. /7/ |
| 8 | nt 502 G→C. exon 3 | HFE E168Q | present invention |
| 9 | nt 502 G→T exon 3 | HFE E168X | present invention |
| 10 | nt 506 G→A exon 3 | HFE W169X | present invention |
| 11 | nt 845 G→A exon 4 | HFE C282Y | Feder et al. /1/ |
| 11 | nt 848 A→C exon 4 | HFE Q283P | Rubini et al. /41/ |
| 12 | nt 84-88insC ex 2 | TFR2 E60X | present invention |
| 13 | nt 515 T A exon 4 | TFR2 M172K | present invention |
| 14 | nt 750 C G exon 6 | TFR2 Y250X | present invention |
| 15 | HFE wild-type codon 53 | | |
| 16 | HFE wild-type codon 59 | | |
| 17 | HFE wild-type codon 63-65 | | |
| 18 | HFE wild-type codon 168-169 | | |
| 19 | HFE wild-type codon 282-283 | | |
| 20 | TFR2 wild-type codon 60 | | |
| 21 | TFR2 wild-type codon 172 | | |
| 21 | TFR2 wild-type codon 250 | | |

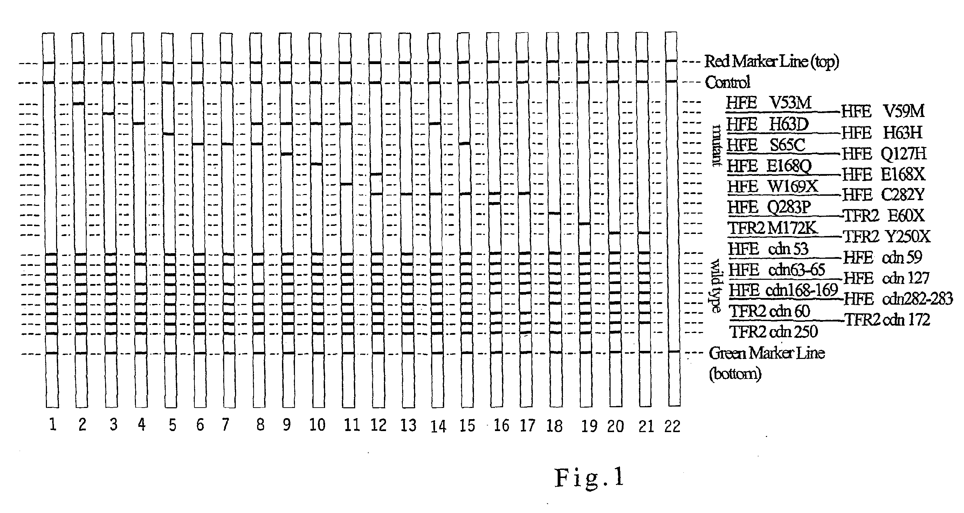

A variety of candidate probes between 16 to 24 nucleotides in length encoding either the wild-type or mutant allele of each mutation were chosen from the GenBank sequences for HFE and TFR2. After enzymatic poly(dT)-tailing the oligonucleotides were applied to a nitrocellulose membrane as an array of parallel lines and fixed by baking. Finally, the membrane sheet was sliced into individual teststrips. Amplification products from different DNA samples covering all 14 mutations were hybridized to these preliminary teststrips under identical and accurately controlled stringency conditions. Bound biotinylated sequences were detected using a streptavidin-enzyme conjugate and color reaction. From these results, the final teststrip was designed by selecting probes which specifically recognized either the wild-type or the mutant allele under the same standardized conditions. A control probe containing biotin residues was included to react positive irrespective of the presence of hybridizing DNA fragments to control the performance of the detection system. The graphic illustration of Fig. 1 shows some possible staining patterns obtainable with DNAs carrying different mutant and wild-type HFE or TFR2 alleles. The following samples were used:
1. non-mutant
2. HFE:V53M heterozygous
3. HFE:V59M heterozygous
4. HFE:H63D homozygous
5. HFE:H63H heterozygous
6. HFE:S65C heterozygous
7. HFE:S65C homozygous
8. HFE:H63D + HFE:S65C compound heterozygous
9. HFE:H63D + HFE:Q127H compound heterozygous
10. HFE:H63D + HFE:E168Q compound heterozygous
11. HFE:HG3D + HFE:W169X compound heterozygous
12. HFE:E168X + HFE:C282Y compound heterozygous
13. HFE:C282Y heterozygous
14. HFE:H63D + HFE:C282Y compound heterozygous
15. HFE:S65C + HFE:C282Y compound heterozygous
16. HFE:C282Y + HFE:Q283P compound heterozygous
17. HFE:C282Y homozygous
18. TFR2:E60X homozygous
19. TFR2:M172K heterozygous
20. TFR2:Y250X heterozygous
21. TFR2:Y250X homozygous
22. negative PCR control

Most of the known techniques are very limited in their capacity to identify more than one mutation in a single run, therefore a comprehensive genetic analysis may become rather labour intensive. On the other hand, highly informative methods such as DNA sequencing are not suitable for routine diagnostic laboratories where high sample throughput is required. With the above described assay the reverse-hybridization technique was successfully applied to analyze 14 different mutations (11 HFE mutations, 3 TFR2 mutations) simultaneously in a single experiment. In the past this method has been extensively used for genotyping of complex hereditary diseases, such as cystic fibrosis /30/ and thalassemia /33/, as well as infectious agents, such as hepatitis C virus /35/.

The staining pattern of a mutant and its corresponding wild-type probe allows the discrimination of three possible genotypes: the mutation is absent if only the wild-type probe stains positive, both signals are visible in heterozygotes, and only the mutant probe is positive in homozygous mutant samples. The same principle applies to the majority of compound heterozygotes for two mutations (Fig. 1, lanes 8-12, 14-16). However, the HFE gene contains at least two regions, one in exon 2 (nt 187-193) and the other in exon 3 (nt 502-506), where several mutations have been found in close proximity. As the hybridization probes span more than one mutation in this area, no wild-type signal is observed if two mutations from the same cluster (e.g. H63D and S65C) occur on different chromosomes in the same sample (Fig. 1, lane 8). The occurance of two neighbouring mutations on a single chromosome would again lead to a different pattern, but has not been described to date.

The reverse-hybridization assay presented here has the potential of becoming a valuable tool for routine diagnosis of multiple HFE and/or TFR2 mutations. By utilizing a single, multiplex amplification reaction and premade, ready-to-use teststrips the procedure is simple, reliable, and can be accomplished within a few hours from blood drawing to final results. For high sample througput, testing can be automated on existing, commercially available equipment, such as the profiBlot II T (TECAN AG, Hombrechtikon, Switzerland). The number of mutations covered by the present assay can easily be extended using the same approach to select additional hybridization probes.

### EXAMPLE 3:

Five Italian probands affected by HH who were heterozygous for C282Y and negative for H63D were studied. They all met the classical phenotypic criteria for homozygous HH:
1) increased transferrin saturation (repeatedly >50% at fast) and increased serum ferritin levels;
2) hepatocellular hemosiderin deposits of grade III or IV according to Scheuer et al. /24/;
3) hepatic iron index ≥ 2.
   Iron loading anemias, B and C chronic viral hepatitis and a history of heavy alcohol intake or of blood transfusions were excluded. All patients and relatives gave their informed consent to the study.

In clinical studies the presence of fibrosis and cirrhosis was determined at liver biopsy. All cirrhotics were in Child's A class. The presence of clinical complications related to HH was determined according to previously described criteria /25/. Transferrin saturation and serum ferritin were measured by standard methods. Hepatic iron concentration (HIC) was determined by atomic absorption spectrophotometry (Perkin-Elmer S2380, Norwak, CT) and the hepatic iron index (HII) (ratio of HIC [µmol/g dry wt] and age [years]) was calculated. The total amount of iron removed by phlebotomy (IR) was estimated as previously reported /25/. The geographical origin of the patients was ascertained up to the fourth generation.

In molecular studies genomic DNA was extracted from peripheral blood leukocytes. C282Y and H63D mutations were detected using standard polymerase chain reaction (PCR) and restriction enzyme digestion with Rsa I and Bcl I, respectively /18/. Microsatellites D6S265, D6S105 and D6S1281 were analysed as described /26/. HLA-A antigens were defined by the microlymphotoxicity test. Haplotypes were constructed manually on the basis of intrafamilial segregation of the marker alleles.

The entire coding sequence and exon-intron boundaries of the HFE gene were amplified by PCR according to Carella et al /18/. The nucleotide sequence of both DNA strands was independently determined in all the probands and in relatives with the C282Y negative chromosome identical to the proband by using the dideoxy chain-termination reaction with Sequenase version 2.0 (USB Amersham, Cleveland,Ohio) and ³⁵S-α-dATP, according to the protocol recommended by the manufacturer. Reactions were separated on a 6% denaturing polyacrylamide gel and visualised by autoradiography. The primers employed for sequencing were the same used for PCR reactions.

To further confirm the presence of the novel mutations (see results section) a restriction length polymorphism analysis (RFLP) was performed. Since the two mutations did not create or destroy a restriction endonuclease site, the portion of exon 3 containing the mutations was amplified by PCR using mismatched sense oligonucleotide specific for each mutation (Bria-F: 5' TGGMCACCAAGCTGGACT 3'; Dom-F: 5' GCCTGGCCCACCAAACTG 3') to create an informative restriction site for BsrSI in the wild type HFE sequence. The antisense oligonucleotide was the same used for sequencing.The results are shown in the following table 2:

**Table 2. Data of the 5 probands affected by hemochromatosis.**

| | Sex | Age yrs | TS % | SF µg/L | Scheuer's grade | HIC µmol/g | HII | IR | Clinical complicat. |
|---|---|---|---|---|---|---|---|---|---|
| 1 | M | 48 | 96 | 694 | 4 | 238 | 4.9 | 4 | none |
| 2 | M | 47 | 79 | 1206 | 4 | 359 | 7.6 | 23 | C |
| 3 | M | 42 | 86 | 608 | 3 | 144 | 3.4 | 3.6 | none |
| 4 | M | 50 | 106 | 2740 | 4 | 425 | 8.5 | 21 | C,H,D |
| 5 | M | 66 | 100 | 1351 | 4 | 421 | 5.4 | - | C,H,A,D |

| | | | | | | | | | |
|---|---|---|---|---|---|---|---|---|---|
| Legend. TS: transferrin saturation; SF: serum ferritin; HIC: hepatic iron concentration; HII: hepatic iron index; IR: iron removed; C: hepatic cirrhosis; H: hypogonadism; A: arthropathy; D: diabetes. | | | | | | | | | |

Table 2 reports iron and clinical data of the 5 probands. Three patients (1, 2 and 3) originated from the same subalpine valley in the north west (Ossola) and two (4 and 5) from an area around the city of Monza (Brianza). Sequence analyses of the HFE gene in probands showed two novel nonsense mutations in exon 3 in the heterozygous state. The first one, a G → T mutation, at the nucleotide 502 of the HFE cDNA sequence (codon 168) (GAG→TAG, Glu→Stop) was found in probands from the Ossola valley. The second, a G → A mutation at nucleotide 506 of HFE cDNA sequence (codon 169) (TGG→TAG, Trp→Stop). was detected in probands from Brianza region. The new variants were named HFE-Ossola and HFE-Brianza from the origin of the patients.

In one family an affected brother who shared the same chromosome 6p haplotypes and the same HFE mutations with the proband was found. His HIC was 176.2 µmol/g and the HII 3.92.

The two new variants were in complete linkage disequilibrium with the C282Y and H63D mutations. HFE-Ossola was associated with haplotype D6S265-3, HLA-A24, D6S105-5 and D6S1281-6 in two families, whereas in the third family, the haplotype changed at D6S265 and HLA-A loci suggesting that a recombination event occurred between HLA-A and D6S105. HFE-Brianza was associated with haplotype D6S265-3, HLA-A24, D6S105-6 and D6S1281-6.

The two mutations determine a stop codon at nucleotide 502 and 506 of the open reading frame (nt 502 GAG → TAG and nt 506 TGG → TAG) respectively, leading to proteins that lack the α3 domain, the transmembrane domain and the cytoplasmic tail. These mutations probably produce, differently from C282Y, a complete disruption of the function of HFE similar to that induced by the partial deletion of exon 4 in HFE-deficient mice /27,28/. Thus, these mutated alleles may behave as a null allele. It is expected that the combination of these mutations in the compound heterozygous state with C282Y leads to severe phenotype expression. Three of the five probands (2, 4 and 5) had indeed a severe disease characterized by the presence of high amount of iron overload and HH-related clinical complications. Proband 3 had a mild phenotype probably due to the fact he was a blood donor for the last 10 years, whereas proband 1 and his affected brother had a relatively mild phenotype and no other obvious protective factors such as blood loss or malabsorption.

Thus, phenotype variability exists in these patients and could be related to both environmental and genetic factors. The existence of modifying genes located around the D6S105 region which influence phenotypic expression of C282Y homozygous HH patients has been recently proposed /25,29/, but further studies are needed to clarify genotype-phenotype correlations in HH patients.

Heterozygotes for HFE-Ossola and HFE-Brianza mutations did not have elevated serum iron indices indicating that neither mutation was able to produce iron overload in the heterozygous state. Moreover, the three compound heterozygotes for either HFE-Ossola or HFE-Brianza and H63D did not show evidence of iron overload. Since they were females and/or young persons, it is possible that, as occur for C282Y, the combination of the two nonsense mutations with H63D or other mild HFE mutations may produce a HH phenotype with low penetrance /17/.

This is the first reported finding of nonsense mutations of the HFE gene. Moreover, this is the first description of several unrelated probands affected by HH who are compound heterozygotes for C282Y and a mutation other than H63D. The two mutations here reported probably originated in the two northern Italian regions and increased in frequency as a result of a local founder effect. Since no consanguinity is present up to the fourth generations between the affected families within each of the two geographical areas, the two mutations should be arisen much more than 100 years ago. Analysis of the frequency of HFE genotypes in HH probands originating from the two Italian regions of Ossola and Brianza shows that compound heterozygotes for C282Y and HFE-Ossola or HFE-Brianza are more frequent than or at least as frequent as C282Y/H63D compound heterozygotes (data not shown). In fact, C282Y/HFE-Ossola compound heterozygotes account for 25% of HH probands in the Ossola region (Vs 8.3% of C282Y/H63D), and C282Y/HFE-Brianza and C282Y/H63D compound heterozygotes each account for 8.4% of HH probands in Brianza region. Whereas the low frequency of C282Y/H63D compound heterozygotes is due to the very low penetrance of this genotype (from 0.44% to 1.5% in different populations) /17/ it is expected that C282Y/HFE-Ossola or HFE-Brianza compound heterozygotes have a 100% penetrance due to the severity of both mutations. These results confirm that HH in Italy is not as homogeneous as in northern Europe and underlines the possibility of other mutations of HFE that may have different relevance in different countries. The recognition of these mutations may have practical implications in diagnostic and screening strategies for HH.

### EXAMPLE 4:

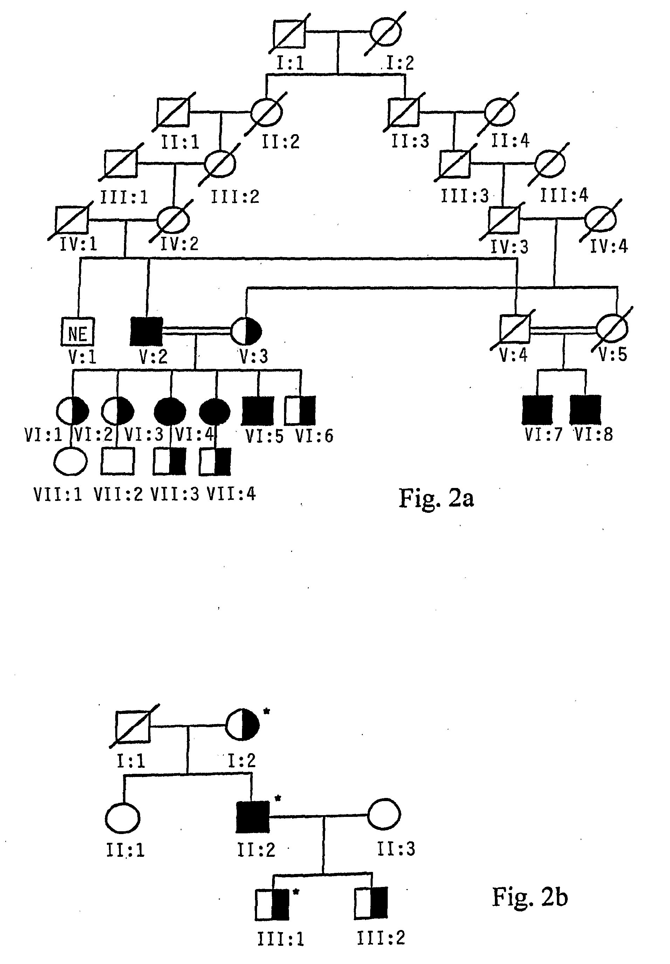

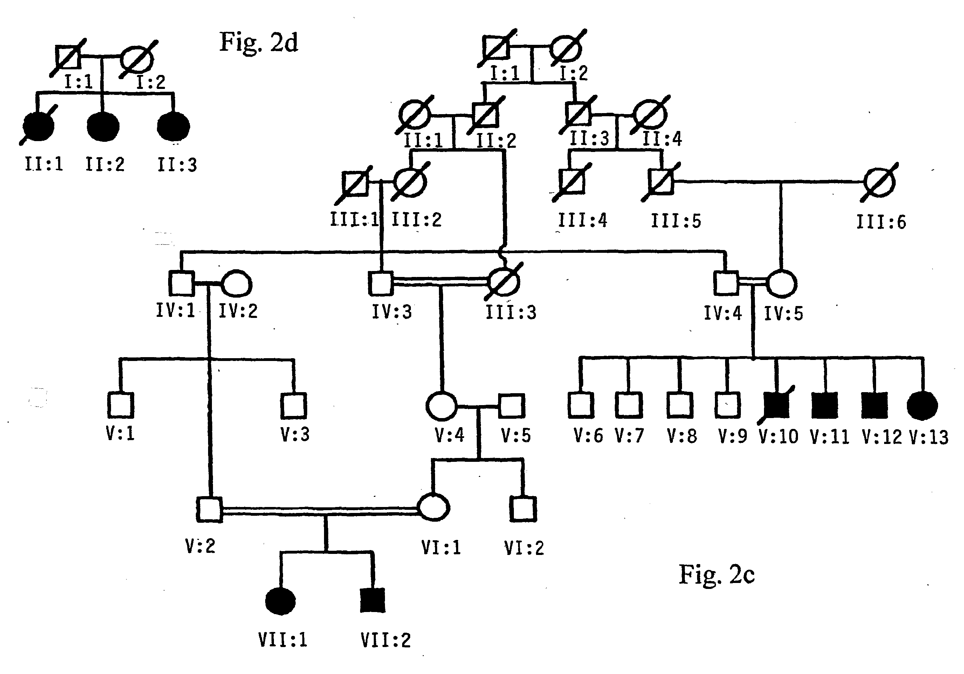

Four different families with hemochromatosis unrelated to HFE were studied. Six patients belong to two Sicilian families (families 1 and 2). The pedigrees of families 1 and 2 are shown in figures 2c and 2d (filled symbols affected, open symbols unaffected, ND=not determined). Four patients belong to a large inbred family (family 3) originating from Campania, a region in southern Italy. The pedigrees of family 3 is shown in figure 2a (filled symbols affected, open symbols unaffected, NE=not examined). A 43 year old man with heavy iron overload, several clinical complications and a wild type HFE genotype belongs to a family (family 4) originated from Lazio in central Italy. The pedigrees of family 4 is shown in figure 2b (filled symbols affected, open symbols unaffected, *=coinheritance of β-thalassemia). Normal subjects were 50 blood donors with normal iron parameters originating from southern Italy.

In molecular studies DNA was prepared from peripheral blood buffy coats or patient's lymphoblastoid cell lines by standard phenolclorophorm extraction /38/. C282Y and H63D mutations in HFE were studied on genomic DNA using PCR-based tests and restriction enzyme digestion with RsaI and MboI (New England Biolabs, Berkeley, MA) respectively /18/. Linkage to the HFE3 locus was established in family 3 analyzing microsatellite D7S651, D7S2498, D7S662, D7S477, D7S1588 allele segregation and two TFR2 intragenic repeats (R1 and R2). Pairwise linkage analysis was performed using the MLINK program from the LINKAGE computer package, as previously described /39/.

PCR was performed in a Thermal Cycler, using 10 pMol of each primer, with an protocol of 32 cycles (denaturation: 94°C 30", annealing: 56°C 45", extension: 72°C 45") and 1 U of AmpliTaq DNApolymerase (Perkin Elmer). PCR primers for TFR2 exon amplification were obtained from databases and are reported in Table 3.

**Table 3. Sequence of TFR2 primers used in the PCR reactions.**

| **Exons** | | **Sequence** | **Fragment lenght** |
|---|---|---|---|
| **exon 1** | F | 5' cctgccaggactgataaggg 3' | 250 bp |
| | R | 5' ggtgggaagaagcgaggtca 3' | |
| **exon 2** | F | 5' tcactgacctcattattgcc 3' | 440 bp |
| | R | 5' aaggctggcgggtggcaaga 3' | |
| **exon 3** | F | 5' gatttctggggtccagaggt 3' | 370 bp |
| | R | 5' aggcagatgggaggactcag 3' | |
| **exon 4** | F | 5' acgtctctggcatccttccct 3' | 276 bp |
| | R | 5' ggtgagcgccccgagccgcg 3' | |
| **exons 5/6** | F | 5' cgcggctcggggcgctcacc 3' | 450 bp |
| | R | 5' cctgaacgattctcactggc 3' | |
| **exons 7/8** | F | 5' ctcctgcccttatgaatcggg 3' | 447 bp |
| | R | 5' gcgggaggcaatgtctgcac 3' | |
| **exon 9** | F | 5' agcgatctggagccagaaag 3' | 350 bp |
| | R | 5' ctatcttgccagggtgtat 3' | |
| **exon 10** | F | 5' gggctgagagacacaggcag 3' | 340 bp |
| | R | 5' caggtacaatgtggatgccg 3' | |
| **exons 11/12** | F | 5' cctcgctaagcctgtcctcc 3' | 325 bp |
| | R | 5' cccagaggcaagggtggacc 3' | |
| **exons 13/14/15** | F | 5' tgagccctgagcctg 3' | 522 bp |
| | R | 5' gccaggacagggtggacgctgg 3' | |
| **exon 16** | F | 5' cccagcgtccaccctgtcctggc 3' | 368 bp |
| | R | 5' ctggattgccagagaggacc 3' | |
| **exon 17** | F | 5' gggtcctggcccctgccgccag 3' | 258 bp |
| | R | 5' aggtccaggaggtggag 3' | |
| **exon 18** | F | 5' cctttgctcccgagccagagcc 3' | 375 bp |
| | R | 5' agcagaggagctcttgactg 3' | |

MaeI enzyme was used to detect Y250X mutant on the amplification product of exon 6. Restriction enzyme analysis of the PCR products was performed according to the manufacturer recommendations.

RNA-SSCP was performed according to previously described protocols /38, 40/. After PCR reaction, transcription was carried out with 10U of T7RNA polymerase in a final volume of 10 µl containing 10mM DTT, 40mM Tris pH 7.5, 6mM MgCl, 2mM spermidine, 10mM NaCl, 5 nmol of each ribonucleoside, 10U of RNAse and 0.2 µl of S35 UTP. After electrophoresis, gels were dried and subjected to autoradiography. Bands showing an electrophoretically altered mobility were directly sequenced. For direct sequencing PCR products were run on 1% agarose gel, purified using QUIAquick PCR purification kit (QIAGEN, Valencia, CA) and sequenced using Thermo Sequenase Cy5.5 dye terminator cycle sequencing kit. After purification from unincorporated dye with Autoseq G-50 columns, sequencing products were electrophoresed in an automatic sequencer (Applied Biosystem 373A) according to the manufacturer's protocols.

Total RNA was prepared by standard methods from peripheral bood buffy coats and retrotranscribed using RNAGeneAmp kit (Roche Molecular System Inc., Branchburg, NJ). Primers used to discriminate α and β transcripts were as reported /37/.

The results in molecular studies showed that no patients were homozygous for C282Y, but patient II-3 of family 2 was homozygous for H63D. After having excluded linkage to HFE2 (juvenile hemochromatosis /26/), it was looked for homozygosity in all effected individuals of both branches of family 1. Pairwise linkage analysis showed a maximum lod score of 4.09 (Θ = 0.0) for markers D7S477 and D7S647 on 7q. This interval was further investigated, confirming the linkage and finding regions of homozygosity of less than 1 cM in both families.

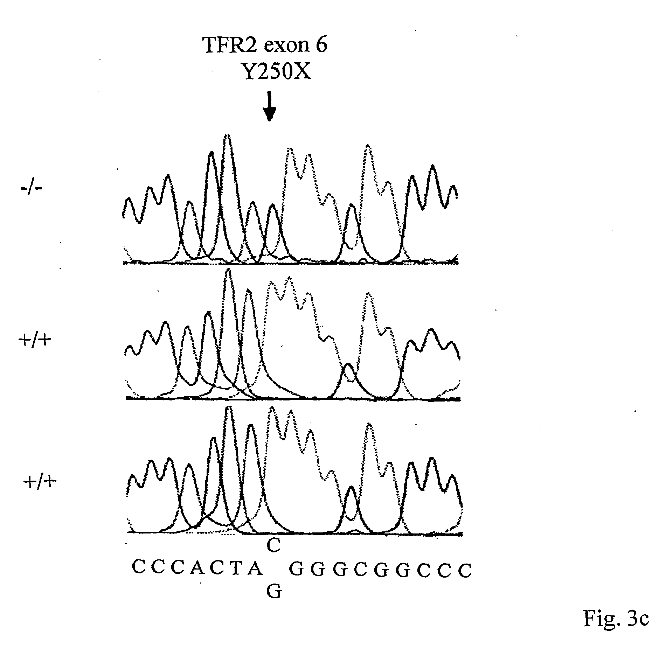

The gene TFR2 was recently isolated and mapped to 7q22 by radiation hybrids /37/. TFR2 shows 66% homology to the transferrin receptor (encoded by TFRC) in its extracellular domain, binds transferrin and is presumed to mediate cellular iron uptake /37/. Using available sequence information, two intragenic polymorphic repeats, (R1 and R2) were identified and homozygosity for these markers was detected in all affected individuals. TFR2 was mapped within the homozygosity region. The TFR2 coding sequence and exon-intron boundaries for mutations were scanned and a C→G transversion in exon 6 at position 750_{.}of the cDNA sequence, which replaces a tyrosine (TAC) with a stop signal (TAG) at residue 250 (Y250X) was detected. Fig.3c shows sequencing chromatographs of the forward sequence of exon 6 spanning the C750G (Y250X) mutation. Subject V-11 (+/+) and VII-1 (+/+) are shown compared to a normal control. The mutation is indicated by an arrow. The Y250X substitution creates a MaeI site. The amplified TFR2 exon 6 was digested with MaeI to analyse segregation of the mutation in family 1. All affected members of family 1 were homozygous for Y250X, whereas obligate carriers were heterozygous. Patient II-3 of family 2 (in which no consanguinity was reported) was homozygous for Y250X. The Y250X mutation was not found in 100 normal chromosomes or in 12 patients, who did not have mutations in HFE.

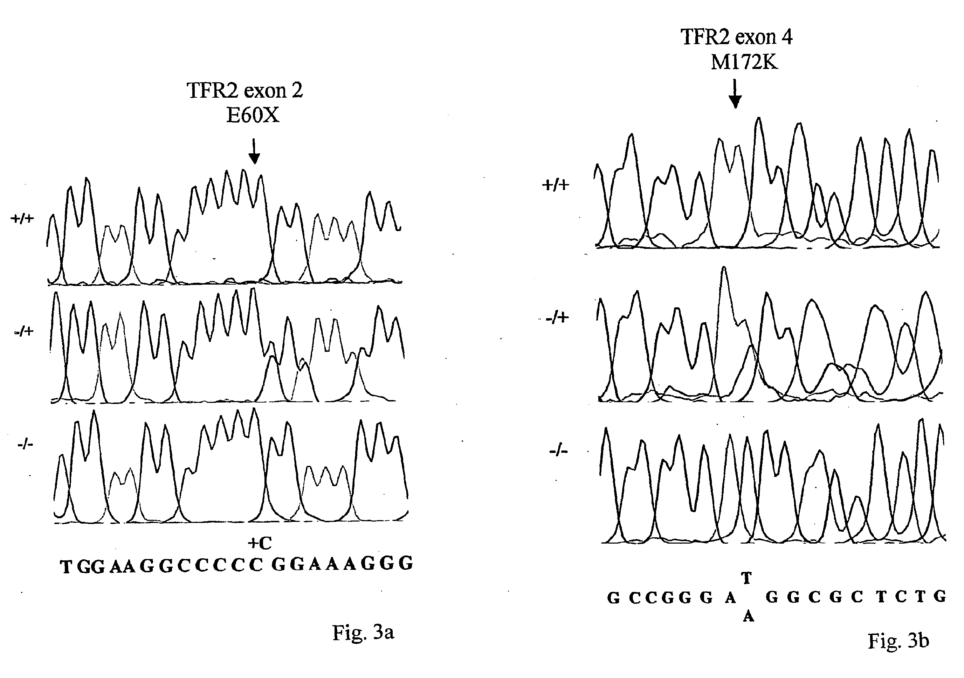

As expected because of consanguinity patients VI-4, VI-5, VI-7 and VI-8 of family 3 were homozygous at TFR2 intragenic repeats and at the other microsatellite markers of 7q (not shown). Based on the principle of homozygosity mapping this finding was suggestive of linkage to this genomic area. When DNA of other family members became available, pairwise linkage analysis was calculated. A lod score of 3.19 at Θ = 0.0 was obtained for the intragenic repeat R1. The whole coding sequence and intron-exon boundaries of TFR2 were scanned for mutations using direct sequencing in subjects VI-7 and VI-8. An insertion of a cytosine at the homozygous state was identified in exon 2 in a polyC tract (84-88 insC). This mutation results in a frameshift followed by a premature stop codon at amino acid 60 (E60X) in the protein. Fig. 3a shows sequencing chromatographs of the forward sequence of exon 2 spanning the C insertion which originates the E60X mutation. Subjects VI-6(+/+) and VI-2(+/-) are shown compared to a normal control (-/-). The position of the insertion is indicated by the arrow. The C insertion was investigated by sequencing DNA of all family members available. Subjects VI-2, VI-3, VI-4 and VI-5 had the mutation at the homozygous state (Table 4) and subjects VI-4, VI-1, VI-2, VI-6, VII-3 and VII-4 had the mutation at the heterozygous state (Table 5). The same mutation was not found in 50 normal DNA samples analyzed by RNA-SSCP.

Patient II-2 of family 4 had a severe disease. HFE2 (juvenile hemochromatosis /26/) was excluded by studying the intrafamily segregation of marker alleles of chromosome 1q, since the patient was haploidentical to a normal sister (not shown). Consanguinity was not reported in this family. However, an homozygous T→A transverion was identified in exon 4 at position 515 of TFR2 cDNA. Fig.3b shows sequencing chromatographs of the forward sequence of exon 4 spanning the T515A (M172K) mutation. Subjects II-2(+/+) and I-2(+/-) are shown compared to a normal control (-/-). The mutation is indicated by the arrow. This nucleotide change results in a lysine for methionine substitution at position 172 of the protein (M172K). T515A segregation within the family was studied by direct sequencing. I-2, III-1 and III-2 were heterozygous for the mutation and II-1 had the normal genotype. The same mutation was not found by RNA-SSCP amoung 50 normal controls.

RT-PCR was performed on total RNA obtained from peripheral blood buffy coats and lymphoblastoid cell lines (LCL) of patients with different TFR2 substitutions and in a normal control. As expected on the bases of the α-transcript restricted pattern of expression, attempts at amplifying the α-transcripts by RT-PCR using RNA from buffy coats or LCL were unsuccessful. Fragments corresponding to the β-transcript were obtained in the normal control and in all patients, except in Y250X homozygotes (not shown).

Clinical data from all HFE3 patients with a defined mutation in TFR2 are summarized in Table 4. No patient was C282Y homozygous or heterozygous. Patient II-3 of family 2 was H63D homozygous. In family 3 a rather variable phenotype was observed: the two probands (VI-7 and VI-8) had a typical disorder with a more severe phenotype in the younger patient. VI-5 had modest liver fibrosis, a remarkable degree of steatosis and increased liver iron staining. The hepatic iron index was borderline (1.99) and approximately 5 g of iron had to be removed to attain iron depletion. VI-3, a 45 yr old female, showed only increased transferrin saturation and serum ferritin. VI-4 and V-2 were undiagnosed and their identification as E60X homozygotes was obtained through family studies (Table 4). VI-4, a premenopausal woman, had remarkably low transferrin saturation and serum ferritin. The finding of iron deficiency without anemia was unexpected in an homozygous mutant subject. Low dietary iron intake and long lasting blood losses through menses in the absence of iron supplementation were identified as the causes of iron deficiency. No history of other blood losses, nor evidence of a hemostasis defect were recorded, but the patient refused a thorough gastrointestinal endoscopic investigation. V-2 had altered iron parameters, abnormal liver function tests and severe arthritis, but never had phlebotomies. His older brother (V-1) was not available for the study, but was reported to be under a regular phlebotomy treatment.

II-2 of family 4 had a severe presentation with cirrhosis, hypogonadism and cardiac disease. As shown in Figure 2b the patient had inherited β-thalassemia at the heterozygous state from his mother.

HFE3 carriers were parents or children of the patients and/or siblings with a documented TFR2 mutation. Clinical data and iron parameters of 15 HFE3 heterozygotes are shown in Table 5. All carriers had normal transferrin saturation and serum ferritin, except V-9 of familiy 1. This subject, who showed increased transferrin saturation and serum ferritin, was affected by HCV chronic hepatitis and underwent occasional phlebotomies. In all the other cases the condition of HFE3 heterozygosity, even in combination with H63D at the heterozygous state (1 case) or with β-thalassemia trait (3 cases), was not associated with iron overload.

This results confirm the role of TFR2 as the HFE3 gene. Scanning for mutations the coding sequence of TFR2 a C insertion (84-88insC), which causes a premature stop codon (E60X) in exon 2, was found at the homozygous state in five affected subjects and in one non-expressing, iron deficient female, from an inbred family. A T515A change in exon 4 that causes a missense (M172K) in the protein, was found at the homozygous state in a single non familial case. The presence of HFE3 homozygotes in two consecutive generations in family 3 witnesses the high degree of inbreeding. The homozygote-heterozygote mating (between V-2 and V-4) explains the absence of wild type TFR2 in the last but one generation, and the seemingly dominant inheritance in this branch of the family.

The identification of 13 subjects with homozygous mutations in TFR2 allows a tentative genotype/phenotype correlation. All classic complications of hemochromatosis were observed among HFE3 patients, although the phenotype severity was variable, especially in family 3. As in HFE-associated disease the intrafamilial variability of the clinical phenotype might be ascribed to both environmental factors and modifier genes. Surprisingly VI-4 of family 3 had iron deficiency without anemia, that was related to heavy menstrual losses and low dietary iron intake. A consistent proportion of C282Y homozygous women are reported not to express the disorder during the fertile age, although iron deficiency is extremely rare both in the literature and in experience.

**Table : 4.Clinical data of HFE3 patients with different TFR2 mutations**

| | Sex | Age at diagnosis | TS | SF | Liver function (HII) | Additional clinical findings | IR | *HFE* mutations | | *TFR2* mutations | | |
|---|---|---|---|---|---|---|---|---|---|---|---|---|
| | | | (%) | (µg/L) | | | (g) | C282Y | H63D | Y250X | E60X | M172K |
| Family 1 | | | | | | | | | | | | |
| V-11 | M | 47 | 100 | 3200 | Cirrhosis | Arthritis | * | *-l-* | *-l-* | +/+ | -/- | -/- |
| V-12 | M | 36 | 100 | 2600 | Abnormal function tests | | * | -/- | -/- | +/+ | -/- | -/- |
| | | | | | | | | | | | | |
| V-13 | F | 45 | 78 | 329 | Abnormal function tests | | * | -/- | -/- | +/+ | -/- | -/- |
| | | | | | | | | | | | | |
| VII-1 | F | 33 | 100 | 2310 | Normal | Hypogonadism | 9.2 | -/- | -/- | +/+ | -/- | -/- |
| VII-2 | M | 27 | 100 | 2930 | Normal | Hypogonadism | 15 | -/- | -/- | +/+ | -/- | -/- |

| Family 2 | | | | | | | | | | | | |
|---|---|---|---|---|---|---|---|---|---|---|---|---|
| II-3 | F | 45 | 100 | 4300 | Cirrhosis | Diabetes Arthritis | * | -/- | +/+ | +/+ | -/- | -/- |

| Family 3 | | | | | | | | | | | | |
|---|---|---|---|---|---|---|---|---|---|---|---|---|
| V-2 | M | 82 | ND | 2550 | Abnormal function | Arthritis | | -/- | -/- | -/- | +/+ | -/- |
| | | | | | tests | | | | | | | |
| VI-3 | F | 45 | 100 | 400 | Normal | | | -/- | -/- | -/- | +/+ | -/- |
| VI-4 ^ | F | 43 | 6 | 6 | Normal | | | -/- | -/- | -/- | +/+ | -/- |
| VI-5 | M | 39 | 100 | 938 | Fibrosis (1.99) | | 4.9 | -/- | -/- | -/- | +/+ | -/- |
| VI-7 | M | 44 | 70 | 1100 | Cirrhosis (2.85) | | 7.2 | -/- | -/- | -/- | +/+ | -/- |
| VI-8 | M | 29 | 78 | 1800 | Cirrhosis | | 24 | -/- | -/- | -/- | +/+ | -/- |

| Family 4 | | | | | | | | | | | | |
|---|---|---|---|---|---|---|---|---|---|---|---|---|
| II-2° | M | 43 | 96 | 2350 | Cirrhosis (11.6) | Hypogonadism | 26 | -/- | -/- | -/- | -/- | +/+ |
| | | | | | | Cardiopathy Arthritis | | | | | | |

| | | | | | | | | | | | | |
|---|---|---|---|---|---|---|---|---|---|---|---|---|
| TS=transferrin saturation; SF=serum ferritin; HII=hepatic iron index; IR=iron removed; ND=not determined *=long term phlebotomies (IR not determined) ^=excessive bleeding through menses °=β-thalassemia trait | | | | | | | | | | | | |

**Table 5.Hematological data and iron parameters of HFE3 carriers with different TFR2 mutations**

| | Sex | Age | TS (%) | SF (µg/L) | Hb . (g/L) | *HFE* C282Y | mutations H63D | Y250X | *TFR2* mutations E60X | M172K |
|---|---|---|---|---|---|---|---|---|---|---|
| Family 1 | | | | | | | | | | |
| V-2 | M | 68 | 36 | 97 | 166 | -/- | -/- | +/- | | |
| V-9 ⁿ | M | 70 | 74 | 310 | 159 | -/- | -/- | +/- | | |
| VI-1 | F | 59 | 27 | 79 | 142 | | | +/- | | |
| * | F | 17 | 38 | 47 | 155 | | | +/- | | |
| * ^ | M | 13 | 25 | 58 | 132 | | | +/- | | |
| * | M | 7 | 34 | 22 | 145 | | | +/- | | |

| Family 3 | | | | | | | | | | |
|---|---|---|---|---|---|---|---|---|---|---|
| V-4 | F | 78 | ND | 32 | ND | -/- | -/- | | +/- | |
| VI-1 | F | 50 | 33 | 19 | ND | -/- | -/- | | +/- | |
| VI-2 | F | 46 | 15 | 30 | 128 | -/- | -/- | | +/- | |
| VI-6 | M | 34 | 26 | 183 | 156 | -/- | -/- | | +/- | |
| VII-3 | M | 27 | 49 | 84 | 147 | -/- | -/- | | +/- | |
| VII-4 | M | 19 | 35 | 33 | ND | -/- | -/- | | +/- | |

| Family 4 | | | | | | | | | | |
|---|---|---|---|---|---|---|---|---|---|---|
| I-2° | F | 72 | 39 | 180 | 112 | -/- | +/- | | | +/- |
| III-1° | M | 18 | 40 | 75 | 132 | -/- | -/- | | | +/- |
| III-2 | M | 16 | 30 | 44 | 140 | -/- | -/- | | | +/- |

| | | | | | | | | | | |
|---|---|---|---|---|---|---|---|---|---|---|
| TS=transferrin saturation; SF=serum ferritin; ND=not determined ⁿ=HCV chronic hepatis *=children of patients VII-1 and VII-2 ^=δβ-thalassemia °=β-thalassemia trait | | | | | | | | | | |

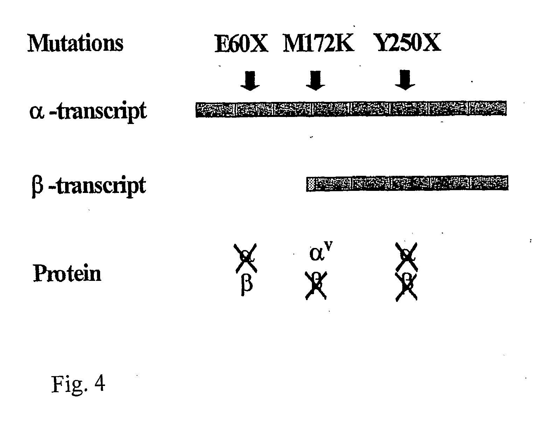

It is hypothesized, that the different effects of the mutations on the encoded protein may influence the phenotype severity. In theory, as shown in Fig. 4, the effect of the reported mutations on TFR2 transcripts is different. Two transcripts possibly resulting from the alternative splicing according to Kawabata et al /37/ are shown in Fig.4. β-transcript lacks exon 1-3 and has additional nucleotides at the 5'end (dotted box) as compared to the α-transcript. The position of the mutations identified and their possible effect on the proteins are shown. α^{V} indicates the variant with the aminoacid change M172K. E60X occurs in exon 2 and disrupts the predicted α-TFR2 transcript, but at variance with Y250X, does not interfere with the β-transcript. T515A causes a missense in the α-protein (M172K). The methionine at position 172 is conserved in the mouse and the substitution of a basic for a neutral aminoacid might change the α-protein properties. However, the same nucleotide substitution affects also the putative initiation codon of the β-variant, preventing its translation. It is speculated that the absence of β-TFR2 might be associated with a severe phenotype in Y250X and M172K homozygotes, while its persistence might be responsible for a partial function of the protein in E60X homozygotes. However, to establish whether β-TFR2 has any physiological significance requires further investigations. At present antibodies against TFR2, which could help to confirm our hypothesis, are not available. Preliminary results of RT-PCR on RNA from peripheral blood or LCL showed the absence of β-TFR2 only in Y250X homozygotes, a result which is in keeping with a rapid degradation of a nonsens transcript. Such a degradation is not expected in M172K, which affects the β-TFR2 initiation codon, neither in E60X, which does not affect the β-transcript.

A contribution of β-thalassemia trait to the phenotype severity cannot be ruled out in II-2 of family 4, as it was observed that the coinheritance of β-thalassemia trait aggravates the phenotype expression in C282Y HFE homozygotes.

Mutations of TFR2 at the heterozygous state were usually well tolerated. As observed in C282Y HFE heterozygous mutants, individuals with heterozygous mutations of TFR2 in association with β-thalassemia trait do not develop iron overload (Table 5).

The results may have practical implications for molecular diagnosis of hemochromatosis. Genotyping the HFE gene is included in the disease diagnostic protocols. However, in all the reported series a minority of patients have wild type or incomplete HFE genotypes (C282Y at the heterozygous or H63D at the heterozygous or homozygous state). They are considered to be affected by atypical forms of hemochromatosis or by secondary iron overload. Our data show that a different non-HFE determinant may be present in these cases, as examplified by patient II-3 of family 2. Therefore screening for mutations TFR2 is a new diagnostic tool that can be offered to patients without HFE mutations or with an incomplete HFE genotype.

Finally, the identification of TFR2 as the HFE3 gene is of relevance to the issue of modifier genes in hemochromatosis. The presence of modifier genes that may modulate (either ameliorate or worsen) the phenotype has been demonstrated in mice and hypothesized in humans. TFR2 is the first obvious modifier to be investigated in C282Y homozygotes.

### References

1. Feder J.N., Gnirke A., Thomas W., Tsuchihashi Z, Ruddy D.A., Basava A., Dormishian F., Domingo R., Ellis M.C., Fullan A., Hinton L.M., Jones N.L., Kimmel B.E., Kronmal G.S., Lauer P., Lee V.K., Loeb D.B., Mapa F.A., McClelland E., Meyer N.C., Mintier G.A., Moeller N., Moore T., MoriKang E., Prass C.E., Quintana L., Starnes S.M., Schatzman R.C., Brunke K.J., Drayana D.T., Risch N.J., Bacon B.R., Wolff R.K.. A novel MHC class I-like gene is mutated in patients with hereditary haemochromatosis. Nat Genet 1996;13:399-408.
2. Jazwinska, E.C., Cullen, L.M., Busfield, F., Pyper, W.R., Webb, S.I., Powell, L.W., Morris, C.P., and Walsh, T.P. (1997). Haemochromatosis and HLA-H. Nature Genet. 14, 249-251.
3. Oberkanins C., Kazemi-Shirazi L., Kury F., Polli C., Maier-Dobersberger T., Yeganehfar C., and Ferenci P. (1997). Genotyping of the hereditary haemochromatosis mutation (HLA-H:C282Y) in microtiter plates. Am. J. Hum. Genet. 61, A225 (Suppl.).
4. Steffensen R., Varming K., and Jersild C. (1998). Determination of gene frequencies for two common haemochromatosis mutations in the Danish population by a novel polymerase chain reaction with sequence-specific primers. Tissue Antigens 52, 230-235.
5. Bollhalder M., Mura C., Landt O., and Maly F.E. (1999). LightCycler PCR assay for simultaneous detection of the H63D and S65C mutations in the HFE hemochromatosis gene based on opposite melting temperature. Clin. Chem. 45, 2275-2278.
6. Christiansen L., Bygum A., Thomsen K., Brandrup F., Horder M. and Petersen N.E. (1999). Denaturing gradient gel electrophoresis analysis of the hemochromatosis (HFE) gene: impact of HFE gene mutations on the manifestation of porphyria cutanea tarda. Clin. Chem. 45, 2025-2026.
7. Villiers J.N., Hillermann R., Loubser L., Kotze M.J. Spectrum of mutations in the HFE gene implicated in haemochromatosis and porphyria. Hum Mol Genet 1999;8:1517-22.
8. Mangasser-Stephan K., Tag C., Reiser A., and Gressner A.M. (1999). Rapid genotyping of haemochromatosis gene mutations on the LightCycler with fluorescent hybridization probes. Clin. Chem. 45, 1875-1878.
9. Mura C., Raguenes O., Ferec C.. HFE mutations analysis in 711 hemochromatosis probands: evidence for S65C implication in mild form of hemochromatosis. Blood 1999;93:2502-5.
10. Miller S.A., Dykes D.D., and Polesky H.F. (1988). A simple salting out procedure for extracting DNA from human nucleated cells. Nucleic Acids Res. 16, 1215.
11. Bacon B.R., Powell L.W., Adams P.C., Kresina T.F., Hoofnagle J.H. Molecular medicine and hemochromatosis: at the crossroads. Gastroenterology 1999;116:193-207.
12. Camaschella C., Piperno A. Hereditary Hemochromatosis: recent advances in molecular genetics and clinical management. Haematologica 1997;82:77-84.
13. Jouanolle A.M., Gandon G., Jezeque P., Blayau M., Campion M.L., Yaouanq Y., Mosser J., Fergelot P., Chauvel B., Bouric P., Carn G., Andrieux N., Gicquel I., Le Gall J.Y., David V. Haemochromatosis and HLA-H. Nat Genet 1996;14:251-2.
14. Beutler E., Gelbert T., West C., Lee P., Adams M., Blackston R., Pockros P., Kosty M., Venditti C.P., Phatak P.D., Seese N.K., Chorney K.A., Ten Elshof A.E., Gerhard G.S., Chorney M. Mutation analysis in hereditary hemochromatosis. Blood Cells Mol Dis 1996;22:187-94.
15. Pietrangelo A., Camaschella C. Molecular genetics and control of iron metabolism in Hemochromatosis. Haematologica 1998;83:456-61.
16. Beutler E. Genetic iron beyond haemocromatosis: clinical effects of HLA-H mutations. Lancet 1997;349:296-7.
17. Beutler E. The significance of 187G (H63D) mutation in hemochromatosis. Am J Hum Genet 1997;61:762-4.
18. Carella M., D'Ambrosio L., Totaro A., Grifa A., Valentino M.A., Piperno A., Girelli D., Roetto A., Franco B., Gasparini P., Camaschella C. Mutation analysis of HLA-H gene in Italian hemochromatosis patients. Am J Hum Genet 1997;60:828-32.
19. Piperno A., Sampietro M., Pietrangelo A., Arosio C., Lupica L., Montosi G., Vergani A., Fraquelli M., Girelli D., Pasquero P., Roetto A., Gasparini P., Fargion S., Conte D., Camaschella C. Heterogeneity of hemochromatosis in Italy. Gastroenterology 1998;114:996-1002.
20. Camaschella C., Fargion S., Sampietro M., Roetto A., Bosio S., Garozzo G., Arosio C., Piperno A. Inherited HFE-unrelated hemochromatosis in Italian families. Hepatology 1999;29:1563-4.
21. Pietrangelo A., Montosi G., Totaro A., Garuti C., Conte D., Cassanelli S., Fraquelli M., Sardini C., Vasta F., Gasparini P. Hereditary Hemochromatosis in adults without pathogenic mutations in the hemochromatosis gene. New Engl J Med 1999;341:725-32.
22. Wallace D.F., Dooley J.S,. Walker A.P. A novel mutation of HFE explains the classical phenotype of genetic hemochromatosis in a C282Y heterozygote. Gastroenterology 1999;116:1409-12.
23. Barton J.C., Sawada-Hirai R., Rothenberg B.E., Acton R.T. Two novel missense mutations of the HFE gene (I105T and G93R) and identification of the S65C mutation in Alabama hemochromatosis probands. Blood Cells Mol Dis 1999;25:146-54.
24. Scheuer P.J., Williams R., Muir A.R. Hepatic pathology in relatives of patients with hemochromatosis. J. Pathol Bacteriol 1962;84:53-64.
25. Piperno A., Arosio C., Fargion S., Roetto A., Nicoli C., Girelli D., Sbaiz L., Gasparini P., Boari G., Sampietro M., Camaschella C.. The ancestral hemochromatosis haplotype is associated with a severe phenotype expression in italian patients. Hepatology 1996;24:43-6.
26. Camaschella C., Roetto A., Ciciliano M., Pasquero P., Bosio S., Gubetta L., Di Vito F., Girelli D., Totaro A., Carella M., Grifa A., Gasparini P. Juvenile and adult hemochramatosis are distinct genetic disorders. Eur J Hum Genet 1997;5:371-5.
27. Zhou X.Y., Tomatsu S., Fleming R.E., Parkkila S., Waheed A., Jiang J., Fei Y., Brunt E.M., Ruddy D.A., Prass C.E., Schatzman R.C., O'Neill R., Britton R.S,. Bacon B.R., Sly W.S. HFE gene Knockout produces mouse model of hereditary hemochromatosis. Proc Natl Acad Sci USA 1998;95:2492-7.
28. Levy J.E., Montross L.K., Cohen D.E., Fleming M.D., Andrews N.C.. The C282Y mutation causing hereditary hemochromatosis does not produce a null allele. Blood 1999;94:9-11.
29. Pratiwi R., Fletcher L.M., Pyper W.R., Do K.A, .Crawford D., Powell L.W., Jazwinska E.C. Linkage disequilibrium analysis in Australian haemochromatosis patients indicates bipartite association with clinical expression. Journal of Hepatology 1999;31:39-46.
30. Chehab F.F., and Wall J. (1992). Detection of multiple cystic fibrosis mutations by reverse dot blot hybridization: a technology for carrier screening. Hum. Genet. 89, 163-168.
31. Douabin V., Moirand R., Jouanolle A.M., Brissot P., Le Gall J.Y., Deugnier Y., and David V. (1999). Polymorphisms in the HFE gene. Hum. Hered. 49, 21-26.
32. Jeffrey G.P., Chakrabarti S., Hegele R.A., and Adams P.C. (1999). Polymorphism in intron 4 of HFE may cause overestimation of C282Y homozygote prevalence in haemochromatosis. Nature Genet. 22, 325-326.
33. Maggio A., Giambona A., Cai S.P., Wall J., Kan Y.W., and Chehab F.F. (1993). Rapid and simultaneous typing of hemoglobin S, hemoglobin C, and seven Mediterranean (-thalassemia mutations by covalent reverse dot-blot analysis: application to prenatal diagnosis in Sicily. Blood 81, 239-242.
34. Saiki R.K., Walsh P.S., Levenson C.H., and Erlich H.A. (1989). Genetic analysis of amplified DNA with immobilized sequence-specific oligonucleotide probes. Proc. Natl. Acad. Sci. USA 86, 6230-6234.
35. Stuyver L., Rossau R., Wyseur A., Duhamel M., Vanderborght B., Heuverswyn H.V., and Maertens G. (1993). Typing of hepatitis C virus isolates and characterization of new subtypes using a line probe assay. J. Gen. Virol.
36. Lebron J.A., Bennett M.J., Vaughn D.E. et al Crystal structure of the hemochromatosis protein HFE and characterization of its interaction with transferrin receptor. Cell. 1998, 93:111-123.
37. Kawabata H., Yang R., Hirama T. et al Molecular cloning of Transferrin receptor 2. J.Biol Chem. 1999, 274:20826-20832.
38. Sambrook J., Frisch E., Maniatis T. Molecular cloning: a laboratory manual, Vol.1-3, Cold Spring Harbor Laboratory Press, Cold Spring Harbor, NY, 1989.
39. Roetto A., Totaro A., Cazzola M. et al The Juvenile Hemochromatosis Locus Maps To Chromosome 1q. Am J Hum Genet. 1999, 64:1388-1393.
40. Sarkar G., Yoon H.S., Sommer S. Screening for mutations by RNA single strand conformation polymorphisms (rSSCP): comparison with DNA-SSCP. Nucleic Acids Res. 1992, 20:871-878.
41. Rubini, M. Carturan S., Barp L., Bononi I., Baricordi O., Europ.J.Hum.Genet. (2000), Vol.8 (Suppl. 1), p.158.

## Claims

1. Method of determining a HFE mutation in a patient, comprising
- providing a biological sample of said carrier, and
- determining a mutation that results in a E168X mutation of HFE.

2. Method according to claim 1, wherein said mutation is determined by identifying a mutation nt502 G→ T.

3. Method according to claim 1 or 2, wherein additional HFE mutations are determined selected from the group consisting of C282Y and/or H63D.

4. Method according to any of claims 1 to 3, wherein further HFE not showing said HFE mutation is determined, to prove whether the mutation is heterozygeous or homozygeous.

5. Method according to any of claims 1 to 4, wherein said mutation is determined using a specific probe hybridizing with the region corresponding to said mutation.

6. Method according to any of claims 1 to 4, wherein said mutation is determined by sequence analysis.

7. Use of a method according to any of claims 1 to 6, to determine if the patient is affected by or a carrier of hemochromatosis.

8. Use according to claim 7, to determine if the patient has a homozygeous or heterozygeous mutation.

9. Sequence specific hybridization probe for use in a method according to any of claims 1 to 5, which is immobilized.

10. Probe according to claim 9, which is immobilized on a test strip, ready-to-use to test isolated DNA.

11. Set of probes for use in a method according to any of claims 1 to 5, containing
- a probe specific for nt 502 G→ T,
and at least one of
- a probe specific for nt 845 G→ A, or
- a probe specific for nt 187 C→ G.

12. Set according to claim 11, which further contains a probe specific for a HFE sequence not showing said mutation.
